# EUROPEAN PATENT APPLICATION

(11) **EP 4 354 062 A1**
(43) Date of publication of application: **17.04.2024**
(21) Application number: 23202105.5
(22) Date of filing: 06.10.2023
(51) Int. Cl.: F26B 21/00

(54) **METHOD OF REMOVING MOISTURE FROM WETTED COMPONENTS, FIXTURES AND FITTINGS TO SANITISE OR PREVENT BACTERIAL GROWTH AND DEVICES FOR USE IN SUCH**

(30) Priority: 10.10.2022 GB 202214918
(71) Applicant: Envirocloud Ltd, Malpas Cheshire SY14 7ES (GB)
(72) Inventor: MAKIN, Thomas, Malpas, SY14 7ES (GB)
(74) Representative: Forresters IP LLP

(57) **Abstract**

The present invention provides a method of removing moisture from wetted components, fixtures, and fittings prior to their installation or reinstallation within a domestic or process water system to prevent the growth of microorganisms within said system. The wetted components, fixtures, and fittings can be wet as a result of liquid based pressure testing carried out on them in post-manufacture quality assurance or they can be used components, fixtures, and fittings that have been removed from an existing water system. The moisture is removed by inducing the movement of a gas through the components, fixtures, and fittings to dry out their internal surfaces. The gas can be blown and/or sucked through the components, fixtures, and/or fittings. The present invention also provides an apparatus for use in removing moisture from wetted components, fixtures, and fittings.

## Description

### Field of the Invention

The present invention relates to methods of removing moisture to prevent microbial growth and/or to sanitise wetted components, fixtures, and fittings prior to their installation or reinstallation in a domestic or process water system. The present invention also relates to apparatus used in such processes.

### Background of the Invention

Domestic and process water systems utilise various types of components, fixtures, and fittings, each of which travels its own manufacture and testing pathway before finally being installed in such systems.

During the testing of components, fixtures, and fittings destined from use in a domestic or process water system, it is not uncommon for them to be subjected to liquid-based testing in order to check their integrity.

One example of a liquid-based test is pressure testing, in which a component, fixture, or fitting is subjected to pressurised fluid for a period of time and its performance is monitored to check for leaks or loss of pressure.

Upon passing these quality control tests, the components, fixtures, and fittings are packaged up and then shipped to a distribution centre for subsequent sale and installation in a domestic or process water system.

It is not uncommon for components, fixtures, and fittings to be held in storage for significant periods of time after they have passed through liquid-based testing.

During this period of storage prior to their installation in a domestic or process water system, these components, fixtures, and fittings, which are wetted as a result of the liquid-based testing, can provide a suitable environment for the growth of colonies of waterborne pathogens, examples of which include Legionella and Pseudomonas.

If these pathogen colonies are allowed to proliferate within a component, fixture, or fitting, the pathogens can reach levels that are sufficient to enable their propagation to the rest of a water system (e.g. domestic or process) following the installation of said component, fixture, or fitting in said system. This can lead to the system-wide proliferation of the pathogen colonies, which can necessitate expensive remedial action, such as chemical flushing.

In addition, contaminated components, fixtures, and fittings can also directly infect `at risk' persons (e.g. a tap supplied to a healthcare environment).

It is not only new, unused fixtures, fittings, and components that can face issued with pathogens. Fixtures, fittings, and components that have already been installed within a water system can also be found to be colonised with bacteria. In cases where traditional remedial actions, such as chemical disinfections, prove unsuccessful the contaminated components, fixtures and fittings are simply thrown away and replaced.

### Summary of the Invention

In the light of the environmental issues associated with the build-up of pathogens, such as bacteria, in wetted components, fixtures and fittings, the present invention provides a method of removing moisture from wetted components, fixtures, and fittings, according to claim 1.

In this regard, there is provided a method of removing moisture from wetted components, fixtures, and/or fittings prior to their installation or reinstallation within a domestic or process water system to prevent the growth of microorganisms within said system; wherein said method comprises inducing the movement of a gas through said components, fixtures, and/or fittings to dry out the internal surfaces thereof; and whereby the gas movement is achieved by blowing and/or sucking the gas through said components, fixtures, and/or fittings.

It is envisaged that by generating a movement of gas through the components, fixtures, and/or fittings before they are installed in a domestic or process water system it is possible to remove any residual moisture that might harbour unwanted waterborne pathogens. The removal of the moisture is achieved by a combination of physically driving it out of the components, fixtures, and/or fittings and evaporating the moisture.

In this way, the components, fixtures, and/or fittings are effectively sanitized before they are attached to the rest of a water system. It will be appreciated that this approach reduces the likelihood of pathogen colonies being introduced into the system when new components, fixtures, and/or fittings are installed.

Preferably, the moisture in the wetted components, fixtures, and/or fittings may be introduced by a water-based pressure testing apparatus. As such, the method of the present invention may be employed shortly after the pressure testing of the new components, fixtures, and/or fittings has been carried out.

It is envisaged that by drying out the interior surfaces of components, fixtures, and/or fittings promptly it is possible to effectively limit the growth of waterborne pathogen colonies; even when said components, fixtures, and/or fittings are placed into long term storage prior to their eventual use.

It is also envisaged that the process of moisture removal can also be effectively applied to older components, fixtures and/or fittings that have already been deployed within a domestic or process water system. In this regard it is appreciated that existing components, fixtures and/or fittings within established water systems can become contaminated with pathogen colonies. Such colonies can prove difficult to remove effectively or efficiently using traditional system-wide approaches (e.g., chemical flushing).

Currently, in situations where the contaminated wetted components, fixtures and/or fittings cannot be effectively sanitized they are simply disposed of and replaced, which can involve considerable cost when multiple pieces are replaced at the same time.

Preferably the method of the present invention may further comprise the step of removing said wetted components, fixtures and/or fittings from a domestic or process water system prior to inducing the movement of gas through said components, fixtures and/or fittings.

Inducing the movement of a gas within the components, fixtures, and/or fittings will not only achieve a drying effect on any moisture within them, but it will also help to desiccate resident organisms and loosen biofilms which makes them easier to extract.

It is appreciated that a range of gases may be suitably used in the method of the present invention. However, preferably the gas may be selected from a group consisting of: air, carbon dioxide, nitrogen, ozone (Os) and other inert gases.

Preferably the movement of the gas within the wetted components, fixtures, and/or fittings may comprise a one-way flow of gas. This could be achieved by sucking or blowing the gas through said wetted components, fixtures, and/or fittings.

Alternatively, the movement of gas within the wetted components, fixtures, and/or fittings may comprise a tidal flow of gas. When a tidal flow approach is used, gas is periodically blown into said wetted components, fixtures, and/or fittings and then sucked out again in a manner similar to that of the human lungs.

Preferably the gas may be heated before it enters the wetted components, fixtures, and/or fittings. In this way the drying effects of the gas as it moves through the components, fixtures, and/or fittings may be increased. Further preferably the temperature of the gas entering the components, fixtures, and/or fittings may be in the range of 1 to 100 degrees C.

Preferably the gas may be dehumidified before it enters the wetted components, fixtures, and/or fittings. Reducing the moisture content of the gas also serves to increase the drying effect of the gas as it moves through the components, fixtures, and/or fittings.

Preferably the gas may be filtered prior to entering the wetted components, fixtures, and/or fittings and/or after it leaves the components, fixtures, and/or fittings. In this way it is possible to prevent contamination or environmental ingress into the components, fixtures, and/or fittings and/or the release into the environment of potentially harmful microorganisms extracted from the wetted components, fixtures, and/or fittings by the gas flow.

Preferably the components, fixtures, and/or fittings may be arranged in series to form a continuous flow path through the interiors of the components, fixtures, and/or fittings and then the gas is pumped/sucked through the continuous flow path to dry out the internal surfaces of the components, fixtures, and/or fittings.

It is envisaged that connecting said components, fixtures, and/or fittings in series so as to form a continuous flow path provides a more efficient way of sanitizing a greater number of components, fixtures, and/or fittings at the same time.

Preferably the method of present invention further comprises the step of housing the components, fixtures, and/or fittings within a drying enclosure, that has a controlled environment, prior to inducing the movement of gas through the components, fixtures, and/or fittings.

Further preferably, the method may further comprises controlling the temperature and/or humidity within the drying enclosure to facilitate the drying out of the gas and/or the components, fixtures, and/or fittings housed therein.

It is envisaged that conducting the method within an environmentally controlled drying enclosure not only helps to make the drying out of the components, fixtures, and/or fittings more efficient, but it also enables any extracted pathogens to be safely contained.

Preferably the method may further comprise the application of a biocide to the components, fixtures, and/or fittings. Further preferably, the biocide may be applied to the components, fixtures and/or fittings before the gas is passed through the components.

Alternatively or additionally, the biocide may be added to the gas before the gas is passed through the components, fixtures, and/or fittings. Alternatively or additionally, the biocide may be applied to the components, fixtures, and/or fittings after the gas has been passed through the components, fixtures, and/or fittings.

Preferably the biocide applied to the components, fixtures, and/or fittings may be ozone (Os) and/ or 70-90% ethyl or isopropyl alcohol.

It is envisioned that using a biocide along with the gas helps to neutralise bacteria within the components, fixtures, and/or fittings at the same time as they are being dried out and extracted by the movement of gas through the components, fixtures, and/or fittings.

In the case of the ethyl or isopropyl alcohol it is envisaged that the alcohol can be delivered as a spray into the components, fixtures, and fittings either before, after, or at the same time as the gas is passed through them.

In addition to acting as a disinfectant, the ethyl or isopropyl alcohol also helps to enhance the drying out process as it evaporates rapidly.

Preferably the components, fixtures, and/or fittings are of the type used in the plumbing system of a building; and wherein the wetted components, fixtures, and/or fittings are selected from the group consisting of taps, showers, valves, and pipes used in plumbing systems.

The present invention also provides an apparatus for removing moisture from the internal surfaces of wetted components, fixtures, and/or fittings prior to their installation or reinstallation within a domestic or process water system, said apparatus comprising: a jig having a gas conduit that provides fluid communication between a primary port and one or more secondary ports, each of which is configured to receive at least one wetted component, fixture, and/or fitting; and gas actuation means connected to the primary port so as to induce the movement of a gas through said secondary ports, such that in use the gas moves through said at least one wetted component, fixture, and/or fitting.

Preferably the gas actuation means may comprise a fan or a pump that is capable of pushing the gas through the primary port via the gas conduit of the jig and out of said secondary ports. In this way gas is blown out of any component, fixture, or fitting engaged on the secondary ports.

Alternatively or additionally, the gas actuation means may comprise a fan or a pump that is capable of drawing the gas through said secondary ports and the gas conduit of the jig and out of the primary port. In this way gas is drawn into and through any component, fixture, or fitting engaged on the secondary ports.

Alternatively, the gas actuation means may comprise a compressed gas source capable of pushing the gas through the primary port via the gas conduit of the jig and out of said secondary ports.

Preferably the apparatus may further comprise a heater to heat the gas being moved through said secondary ports.

Preferably the apparatus may further comprise a dehumidifier to control the humidity of the gas being moved through said secondary ports.

It is envisaged that the apparatus may further comprise an environmentally controlled cabinet. Preferably at least the jig and, in use, the wetted components, fixtures, and/or fittings attached thereto, are housed within an environmentally controlled enclosure (e.g., a cabinet).

Preferably a heater and/or a dehumidifier are employed to control the environment within the cabinet. The internal pressure of the enclosure/cabinet may be maintained at a level greater than the external environment in order to prevent ingress into the enclosure/cabinet.

Preferably the apparatus may further comprise at least one filter configured to filter the gas before it is moved through said secondary ports. Additionally or alternatively, the apparatus may further comprise at least one filter configured to filter the gas before it has moved through said primary port.

It is envisaged that said one or more filters are preferably configured to capture material extracted from the components, fixtures, and/or fittings by the induced movement of gas.

It is envisioned that such material could include harmful organisms and spores that might otherwise be released into the environment when the leave the wetted components, fixtures, and/or fittings.

### Brief Description of the Drawings

The present invention will now be described with reference to drawings, wherein:
Figure 1 shows a diagrammatic view of a first preferred embodiment of an apparatus of the present invention;
Figure 2 shows a diagrammatic view of a second preferred embodiment of an apparatus of the present invention; and
Figure 3 shows a diagrammatic view of a third preferred embodiment of an apparatus of the present invention.

### Detailed Description of the Preferred Embodiments of the Present Invention

It is envisioned that the method of the present invention can be utilized to dry out and effectively sanitize a range of wetted components, fixtures, or fittings prior to their installation or reinstallation in a domestic water system, such as a residential plumbing system, or a process water system.

For example, thermostatic mixing valves, taps or fixtures could be forcefully dried following their performance pressure tests by the manufacturers. Once dry, the valves, fixtures and fittings could be capped and packed ready for dispatch to customer or storage.

Similarly, contaminated components may be removed from a water system and then dried as part of a reactive maintenance regime including cleaning and disinfection in order to eradicate bacteria and other microbiological contamination.

Examples of components, fixtures, or fittings that can be sanitised using the methods and apparatus of the present invention include taps, shower heads, junctions, valves, thermostatic devices, and pipes. However, in the light of the described invention the skilled person will appreciate that any components, fixtures, and/or fittings destined for use in domestic or process water systems that are exposed to liquid during postmanufacture quality control testing would benefit from the present invention.

Components, fixtures, and/or fittings that have been subject to liquid-based testing, one example of which is pressure testing, tend to retain some moisture in and on them.

Also, those components, fixture and/or fittings that have been used with a water system (e.g. a domestic plumbing system) also retain some moisture, which can harbour pathogen colonies.

For the sake of describing the present invention both these components, fixtures, and/or fittings are referred to herein as wetted components, fixtures, and fittings or more simply wetted articles regardless of whether they are 'new' or 'used'.

It will be appreciated that it is the moisture found on the interior surfaces of the wetted articles, which generally contains water, that provides an environment in which certain pathogens, (e.g., bacteria) can flourish overtime.

In the case of new components, fixtures and/or fittings, following their manufacture, these wetted articles can be held in storage for several months before they actually installed in a domestic or process water system. During this time, waterborne pathogens can reproduce to form significant colonies that in turn can migrate to an entire water system once said components, fixtures, and/or fittings are installed.

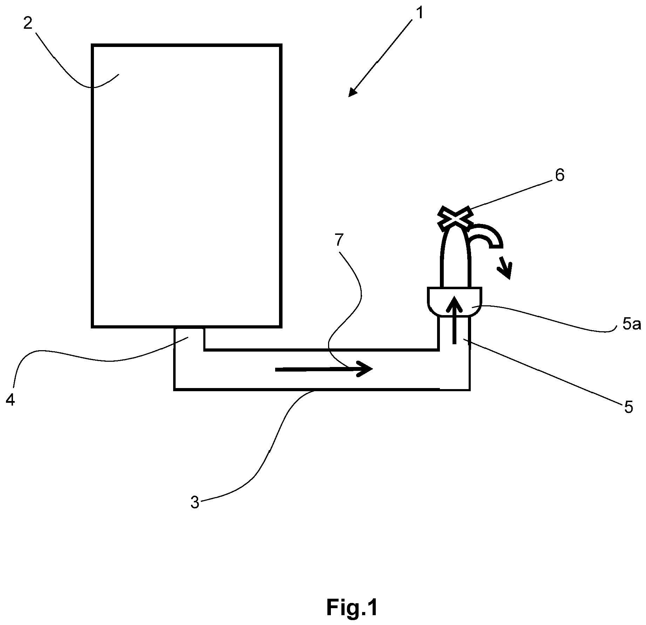

Figure 1 shows a first preferred embodiment of the apparatus 1 of the present invention. The apparatus 1 is designed to remove moisture from one wetted article at a time in order to effectively sanitise it. The component shown in Figure 1 is a tap or faucet 6.

The apparatus 1 shown in Figure 1 comprises a gas actuation means 2 connected in fluid communication with a jig 3 via a primary port 4. The jig 3 is provided with a secondary port 5, which is connected to the primary port 4 by a conduit running through the jig 3.

The secondary port 5 is provided with an attachment means 5a that is configured to form a fluid tight connection with a wetted article (e.g., tap 6) that is engaged with the apparatus 1.

It is envisaged that the attachment means 5a may employ a range of methods to form a fluid tight seal with the wetted article 6.

In one example the attachment means may simply comprise a screw thread that is complementary to that provided on the wetted article. In this way each wetted article can be effectively screwed onto the secondary port before the gas actuation means is operated.

In another example, the attachment means may comprise a simple rubber ring seal mounted in the secondary port 5, into which the wetted article can be inserted. It will be appreciated that, by making the aperture of the rubber ring seal slightly smaller than the portion of the wetted article that is to be received, it is possible to achieve a fluid tight seal between the secondary port 5 and the wetted article 6 when it is inserted into the rubber ring.

Although the quality of the seal formed by the rubber ring arrangement may not be as tight as that provided by the threaded arrangement, it is envisioned that this reduce seal is offset against the speed with which wetted articles can be engaged and disengaged from the apparatus.

Once the wetted article 6 is engaged on the attachment means 5a of the secondary port 5, the gas actuation means 2 is operated to induce the movement of gas through the jig 3 and the received wetted article 6.

In the embodiment shown, the actuation means 2, which may preferably comprise a source of pressurised gas, is operated to push compressed air 7 through the jig 3 via the primary port 4 and the secondary port 5 and into the wetted article 6.

This movement of the air 7 through the system serves to purge the wetted article 6 of any moisture that may have been retained on the interior surfaces of the tap following earlier liquid-based testing (e.g. pressure testing). It is envisaged that forcing compressed air 7 through the wetted article 6 should, over a period of time, both physically drive out the moisture from within the wetted article and also evaporate off (i.e. dry off) the moisture.

With that said, it is envisaged that the speed of evaporation may be increased by forcing heated gas through the wetted article. To this end, the gas actuation means 2 may preferably also be provided with a heater to increase the temperature of the gas 7 before it is passed through the wetted article 6.

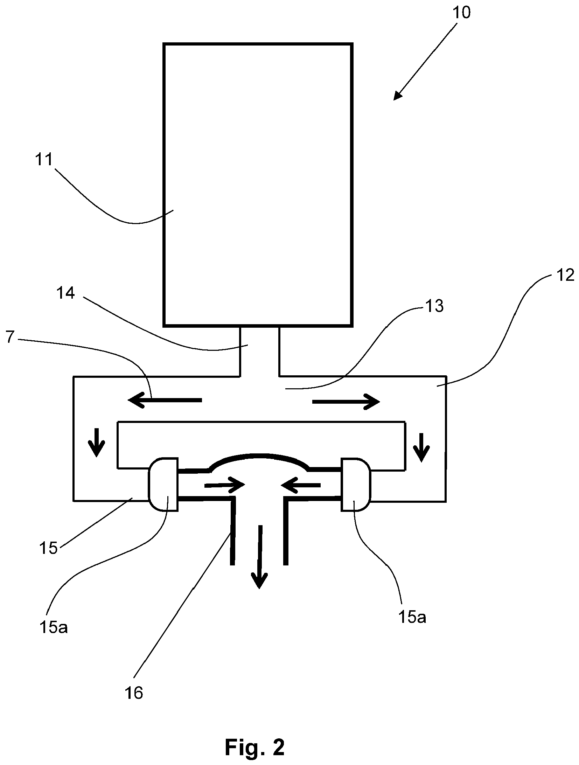

In the case of the tap/faucet 6 shown attached to the apparatus 1 in Figure 1, the gas moves from the inlet of the tap to its outlet along a single pathway. However it is envisaged that some wetted articles may take the form of more complex components with more than one inlet or outlet joined by multiple pathways. The preferred embodiment shown in Figure 2 is an apparatus 10 capable of drying out these more complex types of wetted articles (e.g., mixer valves 16).

The apparatus 10 once again comprises a gas actuation means 11 connected in fluid communication with a jig 12, via the jig's primary port 13. The jig 13 comprises a dual pathway conduit 14 that has a secondary port 15 at the end of each conduit pathway.

As in the apparatus of Figure 1, each secondary port 15 comprises an attachment means 15a that is configured to form a fluid-tight seal with a wetted article. Once again, a screw fit or a push fit connection may be adopted, although the skilled person will appreciate that other suitable connection methods could also be employed to achieve the fluid-tight seal.

Once both of the secondary ports of the jig 12 are engaged with the wetted article 16, the gas actuation means 11 can be operated to purge the moisture from within the wetted article.

The gas actuation means 11 are provided with a fan (not shown) that sucks in air and directs the air 7 through the jig 12 and, in turn, out of the jig's secondary ports 15 into the wetted article 16.

Again, it is envisaged that the speed of the moisture evaporation may be increased by heating the air before it forced through the jig 12 and into the wetted article 16.

Although Figure 2 shows the air 7 flowing from the gas actuation means 11, through the jig and out of the wetted article, it is envisaged that the direction of gas flow may be reversed by operating the fan of the gas actuation means 11 in reverse. In this arrangement the gas is drawn in through the wetted article and then sucked back through the jig 12 to the gas actuation means 11.

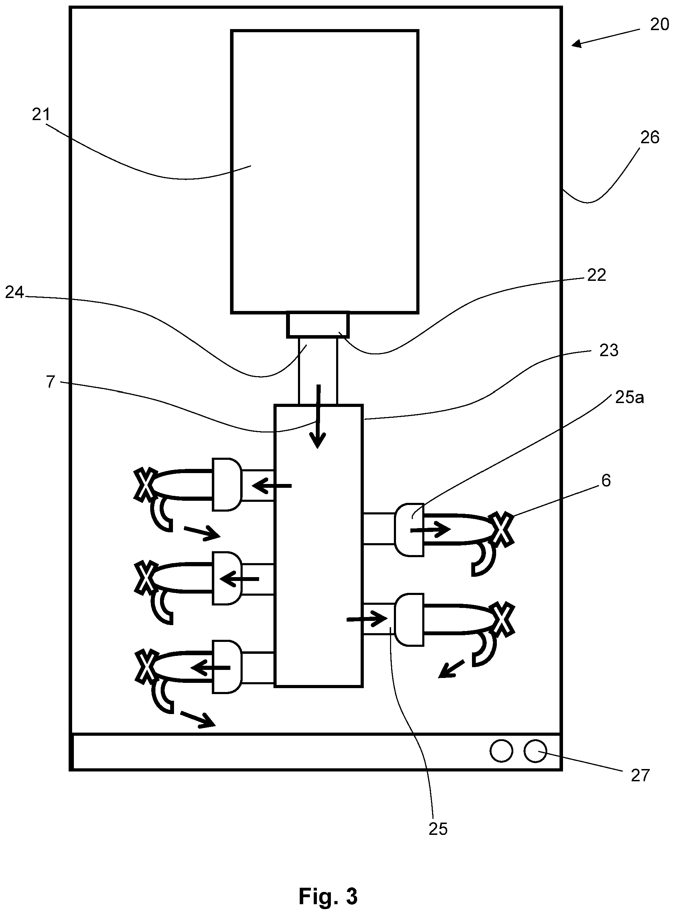

Figure 3 shows a further preferred embodiment of the apparatus 20 of the present invention. The apparatus 20 is designed to engage multiple wetted articles (in this case taps/faucets 6) at the same time so that they can be dried out in batches.

The apparatus 20 is once again provided with a gas actuation means 21 that is connected in fluid communication with an article receiving jig 23. Between the gas actuation means 21 and the primary port 24 of the jig 23 is provided a filter 22. The filter 22 is configured to capture extracted foreign materials (e.g. pathogen biofilms) from the air passing through the system.

Although not shown in the preferred embodiments of Figure 1 and 2, it is envisaged that such apparatus 1, 10 could also include one or more filters without departing from the general concept of the present invention.

In order to accommodate and process multiple wetted articles at the same time, the jig 23 is provided with multiple secondary ports 25. Once again, each port is provided with an attachment means 25a capable of forming a fluid-tight seal with a wetted article.

In the embodiment shown in Figure 3, a tap/faucet 6 is received on each of the secondary ports 25 by virtue of its engagement with a respective attachment means 25a.

Once the taps/faucets 6 are received on the jig 23, the gas actuation means 21 is operated to induce the movement of gas through the jig and the taps engaged thereon.

As note above in relation to the apparatus of Figures 1 and 2, the movement of gas through the system can be achieved in various ways. In the case of apparatus 20, the gas actuation means 21 is proved with a pump which is capable of pushing and/or pulling air through the jig and wetted articles received by the attachment means 25a.

However, it is envisaged that the mechanisms used to induce the movement of gas in each of the disclosed embodiments of the present invention could be interchanged without departing from the general scope of the present invention. Provided, that is, the rate of the gas flow through the wetted articles is maintained at or above a level sufficient to have a drying effect.

Returning to the apparatus 20 of Figure 3, it will be appreciated that the gas actuation means 21, the jig 23 and the wetted articles 6 are all housed within a cabinet 26. The cabinet 26 is sealed so that a controlled environment can be created within it.

It is envisaged that operating the drying out process within a controlled environment speeds up the operation by allowing the process to take place in hotter and/or more humid conditions.

In addition, it ensures that any pathogens purged from the wetted articles during the drying out process are captured for safe disposal. It is appreciated that the provision of the filter 22 is of particular benefit here, as the air within the cabinet 26 may be cycled through the jig and the wetted articles multiple times during the moisture removal process.

The filter 22 acts to prevent purged material from being reintroduced back into the wetted articles as the air flow 7 recycles through them.

Although not shown, it is envisaged that the cabinet 26 may be provided with a heating means and/or dehumidifying means that can be adjusted to achieve an optimum environment for drying out the wetted articles. The cabinet 26 is provided with controls 27 to allow the temperature and humidity levels with the cabinet to be monitored and changed as required.

The present invention will now be described by way of reference to the following clauses:

### Clauses

1. Method of removing moisture from wetted components, fixtures, and fittings prior to their installation or reinstallation within a domestic or process water system to prevent the growth of microorganisms within said system;
   wherein said method comprises inducing the movement of a gas through said components, fixtures, and fittings to dry out the internal surfaces thereof; and
   whereby the gas movement is achieved by blowing and/or sucking the gas through said components, fixtures, and/or fittings.
2. The method of clause 1, wherein the moisture in the wetted components, fixtures and/or fittings is introduced by a water-based pressure testing apparatus.
3. The method of clause 1, further comprising the step of removing said wetted components, fixtures, and/or fittings from a domestic or process water system prior to inducing the movement of gas said components, fixtures, and/or fittings.
4. The method of clause 1, 2 or 3, wherein the gas is selected from a group 15 consisting of air, carbon dioxide, nitrogen, ozone (Os) and other inert gases.
5. The method of clause 1, 2, 3 or 4, wherein the gas movement is applied in one direction through the wetted components, fixtures, and/or fittings.
6. The method of clause 1, 2, 3 or 4, wherein the gas movement is applied in a tidal flow into and out of the components, fixtures, and/or fittings.
7. The method of any one of the preceding clauses, wherein the gas is heated and/or dehumidified before being passed through the components, fixtures, and/or fittings.
8. The method of any one of the preceding clauses, wherein the gas is filtered before and/or after being passed through the components, fixtures, and/or fittings.
9. The method of any one of the preceding clauses, wherein the components, fixtures, and/or fittings are arranged in series to form a continuous flow path through the interiors of the components, fixtures, and/or fittings and then the gas is pumped/sucked through the continuous flow path to dry out the internal surfaces of the components, fixtures, and/or fittings.
10. The method of any one of the preceding clauses, further comprising the step of housing the components, fixtures, and/or fittings within a drying enclosure, that has a controlled environment, prior to inducing the movement of gas through the components, fixtures, and/or fittings.
11. The method of clause 10, further comprising controlling the temperature and/or humidity within the drying enclosure to facilitate the drying out of the gas and/or the 10 components, fixtures, and/or fittings housed therein.
12. The method of any one of the preceding clauses, further comprising the application of a biocide to the components, fixtures, and/or fittings.
13. The method of clause 12, wherein the biocide is:
   a) applied to the components, fixtures, and/or fittings before the gas is passed through the components;
   b) added to the gas before the gas is passed through the components, fixtures, and/or fittings;
   c) applied to the components, fixtures, and/or fittings after the gas has been passed therethrough.
14. The method of clause 12 or 13, wherein the biocide applied to the components, fixtures, and/or fittings is selected from ozone (Os) and/or 70-90% ethyl or isopropyl alcohol.
15. The method of any one of the preceding clauses, wherein the domestic or process water system is the plumbing system of a building; and
   wherein the wetted components, fixtures, and/or fittings are selected from the group consisting of taps, shower heads, junctions, valves, and pipes used in plumbing systems.
16. An apparatus for removing moisture from the internal surfaces of wetted components, fixtures, and/or fittings prior to their installation or reinstallation within a domestic or process water system, said apparatus comprising:
   a jig having a gas conduit that provides fluid communication between a primary port and one or more secondary ports, each of which is configured to receive at least one wetted component, fixture, or fitting; and
   gas actuation means connected to the primary port so as to induce the movement of a gas through said secondary ports, such that in use the gas moves through said at least one wetted component, fixture, or fitting.
17. The apparatus of clause 16, wherein the gas actuation means comprises a fan or a pump that is capable of pushing the gas through the primary port via the gas conduit of the jig and out of said secondary ports.
18. The apparatus of clause 16 or 17, wherein the gas actuation means comprises a fan or a pump that is capable of drawing the gas through said secondary ports and the gas conduit of the jig and out of the primary port.
19. The apparatus of clause 16, wherein the gas actuation means comprises a compressed gas source capable of pushing the gas through the primary port via the gas conduit of the jig and out of said secondary ports.
20. The apparatus of any one of clauses 16 to 19, further comprising a heater to heat the gas being moved through said secondary ports.
21. The apparatus of any one of clauses 16 to 21, further comprising a dehumidifier to control the humidity of the gas being moved through said secondary ports.
22. The apparatus of any one of clauses 16 to 21, wherein at least the jig and, in use, the wetted components, fixtures and/or fittings attached thereto, are housed within an environmentally controlled cabinet.
23. The apparatus of any one of clauses 16 to 22, further comprising at least one filter configured to filter the gas before it is moved through said secondary ports.
24. The apparatus of any one of clauses 16 to 23, further comprising at least one filter configured to filter the gas before it has moved through said primary port.

## Claims

1. Method of removing moisture from wetted components, fixtures, and fittings prior to their installation or reinstallation within a domestic or process water system to prevent the growth of microorganisms within said system;
wherein said method comprises inducing the movement of a gas through said components, fixtures, and fittings to dry out the internal surfaces thereof; and
whereby the gas movement is achieved by blowing and/or sucking the gas through said components, fixtures, and/or fittings.

2. The method of claim 1, wherein the moisture in the wetted components, fixtures and/or fittings is introduced by a water-based pressure testing apparatus.

3. The method of claim 1, further comprising the step of removing said wetted components, fixtures, and/or fittings from a domestic or process water system prior to inducing the movement of gas said components, fixtures, and/or fittings.

4. The method of claim 1, 2 or 3, wherein the gas is selected from a group consisting of air, carbon dioxide, nitrogen, ozone (O₃) and other inert gases.

5. The method of claim 1, 2, 3 or 4, wherein the gas movement is either:
applied in one direction through the wetted components, fixtures, and/or fittings; or
applied in a tidal flow into and out of the components, fixtures, and/or fittings.

6. The method of any one of the preceding claims, wherein:
the gas is heated and/or dehumidified before being passed through the components, fixtures, and/or fittings; and/or
the gas is filtered before and/or after being passed through the components, fixtures, and/or fittings.

7. The method of any one of the preceding claims, wherein the components, fixtures, and/or fittings are arranged in series to form a continuous flow path through the interiors of the components, fixtures, and/or fittings and then the gas is pumped/sucked through the continuous flow path to dry out the internal surfaces of the components, fixtures, and/or fittings.

8. The method of any one of the preceding claims, further comprising the step of housing the components, fixtures, and/or fittings within a drying enclosure, that has a controlled environment, prior to inducing the movement of gas through the components, fixtures, and/or fittings; and
preferably further comprising controlling the temperature and/or humidity within the drying enclosure to facilitate the drying out of the gas and/or the components, fixtures, and/or fittings housed therein.

9. The method of any one of the preceding claims, further comprising the application of a biocide, that is preferably selected from ozone (Os) and/or 70-90% ethyl or isopropyl alcohol, to the components, fixtures, and/or fittings; and
wherein preferably the biocide is:
a) applied to the components, fixtures, and/or fittings before the gas is passed through the components;
b) added to the gas before the gas is passed through the components, fixtures, and/or fittings;
c) applied to the components, fixtures, and/or fittings after the gas has been passed therethrough.

10. The method of any one of the preceding claims, wherein the domestic or process water system is the plumbing system of a building; and
wherein the wetted components, fixtures, and/or fittings are selected from the group consisting of taps, shower heads, junctions, valves, and pipes used in plumbing systems.

11. An apparatus for removing moisture from the internal surfaces of wetted components, fixtures, and/or fittings prior to their installation or reinstallation within a domestic or process water system, said apparatus comprising:
a jig having a gas conduit that provides fluid communication between a primary port and one or more secondary ports, each of which is configured to receive at least one wetted component, fixture, or fitting; and
gas actuation means connected to the primary port so as to induce the movement of a gas through said secondary ports, such that in use the gas moves through said at least one wetted component, fixture, or fitting.

12. The apparatus of claim 11, wherein the gas actuation means comprises a fan or a pump that is capable of pushing the gas through the primary port via the gas conduit of the jig and out of said secondary ports; and
wherein preferably the gas actuation means comprises a compressed gas source capable of pushing the gas through the primary port via the gas conduit of the jig and out of said secondary ports.

13. The apparatus of claim 11, wherein the gas actuation means comprises a fan or a pump that is capable of drawing the gas through said secondary ports and the gas conduit of the jig and out of the primary port.

14. The apparatus of any one of claims 11 to 13, further comprising at least one of:
a) a heater to heat the gas being moved through said secondary ports;
b) a dehumidifier to control the humidity of the gas being moved through said secondary ports;
c) at least one filter configured to filter the gas before it is moved through said secondary ports; and
d) at least one filter configured to filter the gas before it has moved through said primary port.

15. The apparatus of any one of claims 11 to 14, wherein at least the jig and, in use, the wetted components, fixtures and/or fittings attached thereto, are housed within an environmentally controlled cabinet.
